# EUROPEAN PATENT SPECIFICATION

(11) **EP 1 797 063 B1**
(45) Date of publication and mention of the grant of the patent: **05.05.2010**
(21) Application number: 05787206.1
(22) Date of filing: 06.09.2005
(51) Int. Cl.: C07D 333/08, C07D 333/22, A23L 1/226

(54) **USE OF 3-SUBSTITUTED THIOPHENES AS ODORANTS AND FLAVOURINGS**
VERWENDUNG VON 3-SUBSTITUIERTEN THIOPHENEN ALS DUFT- UND AROMASTOFFE
UTILISATION DE TIOPHENES SUBSTITUES EN POSITION 3 COMME AGENTS ODORANTS ET AROMATISANTS

(30) Priority: 10.09.2004 US 609035 P
(43) Date of publication of application: 20.06.2007
(73) Proprietor: Symrise GmbH & Co. KG, 37603 Holzminden (DE)
(72) Inventor: OTT, Frank, 37603 Holzminden (DE); VAN DEN BOS, Christiaan, 37671 Fürstenau (DE); KINDEL, Günter, 37671 Höxter (DE); KRAMMER, Gerhard, 37603 Holzminden (DE); SCHMIDT, Claus-Oliver, 37603 Holzminden (DE)
(74) Representative: Eisenführ, Speiser & Partner
(86) International application number: PCT/EP2005/054375
(87) International publication number: WO 2006/027350

(56) References cited:
- US-A- 3 931 246
- US-A- 3 976 802

## Description

The invention relates to the use of 3-(alkylthiomethyl)thiophenes or 3-(acylthiomethyl)thiophenes as an odorant and flavouring. The invention also relates to preparations, semi-finished products and odorant, flavouring and taste compositions containing the thiophenes according to the invention or to be used according to the invention.

The taste characteristics of cooked meat, mainly beef, lamb, chicken and pork, are characterised by a series of substituted thiazolines, thiazoles, thiapyranes and thiophenes. These compounds are produced by the cooking process. For the formation of these heterocycles, primarily Maillard-like reactions between fats or fat degradation products on the one hand and amino acids on the other hand should be mentioned (ACS Symposium Series 2002, 826, 93-101).

Process flavours, in which fatty acid (e.g. linoleic acid, linolenic acid, oleic acid etc.) react preferably with sulfurous amino acids (cysteine or methionine), also yield the above-mentioned classes of compounds.

Among the thiapyranes and thiophenes, as a result of the autoxidation of the unsaturated fatty acids and of the formation mechanism of the thiapyranes and the thiophenes, substantially 2-alkylated derivatives are obtained. Thus, in various types of cooked meats, different concentrations of 2-ethyl-, 2-propyl-, 2-butyl-, 2-pentyl- and 2-hexyl derivatives of these heterocycles are found (cf. ACS Symposium Series 2002, 826, 93-101).

Thiophene derivatives in which the substituent in the 2-position has a sulfur atom have also undergone sensory investigation. J. Agric. Food Chem. 2003, 51, 3629-3635 may be mentioned as an example. The authors describe 2-thiophenylmethanethiol with the attributes meaty, roasted and grilled, while 3-methyl-2-thiophenylmethanethiol is described as tasting roasted and of coffee. Both compounds were obtained by reacting conjugates of cysteine and thiophene-2-carbaldehyde or cysteine and 3-methylthiophene-2-carbaldehyde with bakers' yeast.

3-(Methylthiomethyl)thiophene as a substance is described in Can. J. of Chem. 1999, 77(4), 463-471). According to J. Am. Chem. Soc. 1993, 115(24), 11608-11609), the corresponding lithiated anion of 3-methylthiomethylthiophene was obtained from 2-bromo-3-(methylthiomethyl)thiophene by metal-halogen exchange, and was further reacted to form thiophene polymers. The sensory properties of 3-(methylthiomethyl)thiophene are not described in any of these documents.

In in-house investigations, it has surprisingly been found that 3-(methylthiomethyl)thiophene and structurally similar 3-(alkylthiomethyl)thiophenes or 3-(acylthiomethyl)thiophenes of the following formula exhibit completely different sensory properties from the above-mentioned (J. Agric. Food Chem., *loc cit.*) 2-substituted thiophenes.

The invention primarily relates, therefore, to the use of a 3-(alkylthiomethyl)thiophene or 3-(acylthiomethyl)thiophene of formula I 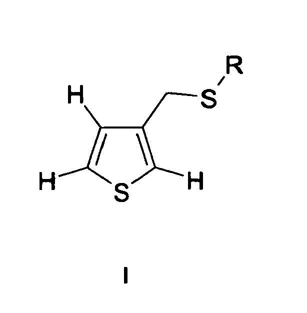 wherein
R represents a C₁ - C₄ alkyl or a C₁ - C₅ acyl residue
as an odorant or flavouring.

The invention also relates to the novel compounds of the above formula I, wherein R represents a C₂ - C₄ alkyl or a C₁ - C₅ acyl residue.

The following table gives the sensory properties of preferred thiophene derivatives according to the invention or to be used according to the invention, established in in-house investigations. The following conditions were selected for in-house tastings: substance to be tasted approximately 300 ppb to approximately 100 ppm, each in a 5% sugar solution and in a 0.5% salt solution.

The strength of each impression is graded from 1 (very weak) to 9 (very strong) :

| R | Sensory properties |
|---|---|
| Methyl | tenacious (8), intensity (8), impact (8), sulfurous (8), leek (7), cress (7), pungent (7), shiitake (6), onion (6), horseradish (5), mustard (5), rubber (4) |
| Ethyl | tenacious (6), intensity (6), impact (6), sulfurous (6), onion (4), leek (4), cress (3), rubber (3) |
| 1-Propyl | tenacious (7), intensity (5), impact (5), leek (4), sulfurous (4), cress (3), onion (3), rubber (3) |
| Formyl | tenacious (7), sulfurous (5), roasted (5), mocha (4), coffee (3), meat (3), metallic (3), onion (2) |
| Acetyl | tenacious (9), mocha (5), sulfurous (5), metallic (5) roasted (5), garlic (4), coffee (3), meat (3), onion (3) |
| Propionyl | tenacious (9), sulfurous (6), roasted (5), garlic (4), onion (3), metallic (2), mocha (2) |

The compounds with longer alkyl or acyl residues (C₄-alkyl or C₄-C₅ acyl residues) have sensory properties similar to those of the compounds with shorter alkyl or acyl residues, but also exhibit fried-bacon and fatty notes (particularly the compounds with an isobutyl and isobutyryl or 3-methylbutyryl residues).

Suitable alkyl residues R are: methyl, ethyl, 1-propyl, 2-propyl, 1-butyl, 2-butyl, 2-methyl-1-propyl- and 2-methyl-2-propyl. The residues methyl, ethyl, 1-propyl and 2-propyl are preferred, and the methyl residue is particularly preferred.

Advantageous acyl residues R are: formyl, acetyl, propionyl, butyryl, 2-methylpropionyl, valeryl, 2-methylbutyryl, 3-methylbutyryl. The residues formyl, acetyl, propionyl, butyryl and 2-methylpropionyl are preferred, and the acetyl residue is particularly preferred.

3-(Methylthiomethyl)thiophene (compound of formula I with R = CH₃) is particularly preferred especially because it possesses the distinctive and thoroughly pungent notes of leek and cress, but also combines strong shiitake and onion notes as well as powerful horseradish and mustard aspects. This compound has very complex sensory properties and is particularly versatile.

The thiophenes according to the invention or to be used according to the invention can advantageously be used together with other flavourings for the production of flavour compositions. Mixtures and combinations of the thiophenes according to the invention or to be used according to the invention can also be used in flavour compositions. The use in the foodstuffs sector of the thiophenes according to the invention or to be used according to the invention is novel.

A flavour composition according to the invention comprises
- a sensorially active quantity of one or more different compounds of formula I 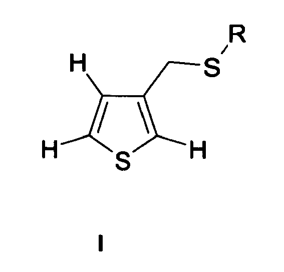
wherein
R represents a C₁ - C₄ alkyl or a C₁ - C₅ acyl residue, and
- a sensorially active quantity or one or more other flavourings, wherein
   the sensorially active quantity or the one or of the different compounds of formula I is selected such that the sensory impression created by the sensorially active quantity of the one or more other flavourings is enhanced and/or modified and/or rounded off.

Flavour compositions according to the invention contain, in addition to at least one thiophene according to the invention or to be used according to the invention, one or more other sensorially active substances, such as e.g. synthetic, natural or nature-identical flavourings or plant extracts. The quantitative ratio of thiophene according to the invention or to be used according to the invention and the other components of a flavour composition is naturally strongly dependent on the desired overall sensory impression of the flavour composition.

The invention also relates to a preparation to be consumed for nutrition or pleasure, containing
(a) a sensorially active quantity or one or more different compounds of formula I 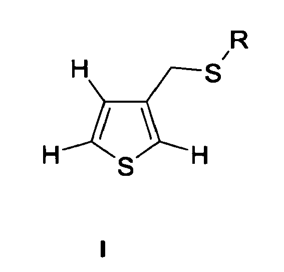 wherein
   R represents a C₁ - C₄ alkyl or a C₁ - C₅ acyl residue or
(b) a flavour composition according to the invention.

As a result of the thiophenes according to the invention or to be used according to the invention, flavour compositions and preparations containing these compounds surprisingly experience additive, more balanced taste impressions, gain extra body and obtain a more natural, more complex and more authentic taste profile.

In addition, it is a great advantage that the thiophenes according to the invention or to be used according to the invention are mostly extraordinarily intensive, tenacious and complex. They therefore go a very long way, can be used in very low doses (in some cases in the ppb range in the preparations) and have a long-lasting action.

It has also been found that the thiophenes according to the invention or to be used according to the invention are not only capable of adding valuable and special taste notes to a flavour composition. In many cases, in flavour composition containing the thiophenes according to the invention or to be used according to the invention, a flavour-intensifying effect (booster or enhancer) is observed, which expresses itself as a generally stronger, and often more rounded, taste impression.

The thiophenes according to the invention or to be used according to the invention are particularly suitable for incorporation in flavour compositions and preparations with the flavour directions of meat, fish or seafood, particularly pork, beef, lamb, chicken, shrimp, prawn, crab and squid. Their use is especially advantageous in flavour compositions and preparations in which pungent, hot notes play a particular role, such as e.g. those with leek, shallot, onion, garlic, cress, horseradish and/or mustard notes.

Preparations according to the invention consumed for nutrition or pleasure generally contain 0.0000001 wt.% (0.001 ppm = 1 ppb) to 0.05 wt.% (500 ppm), preferably 0.0000001 wt.% (0.001 ppm = 1 ppb) to 0.005 wt.% (50 ppm), particularly preferably 0.0000002 wt.% (0.002 ppm = 2 ppb) to 0.001 wt.% (10 ppm) of the thiophenes according to the invention or to be used according to the invention, based on the total weight of the preparation. Other conventional basic, auxiliary and additive substances for foodstuffs, beverages or tobacco can be contained in quantities of up to 99.999999 wt.%, preferably 10 to 80 wt.%, based on the total weight of the preparation. In addition, the preparations can contain water in a quantity of up to 99.999999 wt.%, preferably 5 to 80 wt.%, based on the total weight of the preparation.

The preparations consumed for nutrition or pleasure within the meaning of the invention are e.g. baked goods (e.g. bread, dry biscuits, cakes, other baked products), confectionery (e.g. chocolates, fruit gums, hard and soft toffees, chewing gum, liquorice), alcoholic or non-alcoholic drinks (e.g. coffee, tea, wine, wine-containing drinks, beer, beer-containing drinks, liqueurs, spirits, brandies, fruit-containing fizzy drinks, isotonic drinks, soft drinks, nectars, fruit and vegetable juices, fruit or vegetable juice preparations), instant drinks, meat products (e.g. ham, fresh sausage or dry sausage preparations), eggs or egg products (dried egg, egg white, egg yolk), cereal products (e.g. breakfast cereals, muesli bars), milk products (e.g. milk drinks, dairy ice-cream, yoghurt, kefir, curd cheese, soft cheese, hard cheese, dried milk powder, whey, butter, buttermilk), fruit preparations (e.g. jams, fruit ice, fruit sauces), vegetable preparations (e.g. ketchup, sauces, dried vegetables), snack items (e.g. baked or deep-fried potato crisps or potato dough products, corn- or peanut-based extrudates), fat- and oil-based products or emulsions thereof (e.g. mayonnaise, remoulade, dressings), ready meals and soups, spices, seasoning mixes and particularly seasonings used in the snacks sector. The preparations within the meaning of the invention can also be used as semi-finished products for the production of other preparations consumed for nutrition or pleasure. The preparations within the meaning of the invention can also take the form of capsules, tablets (uncoated as well as coated tablets, e.g. enteric coatings), dragees, granules, pellets, mixtures of solids, dispersions in liquid phases, as emulsions, as powders, as solutions, as pastes or as other preparations for swallowing or chewing as food supplements.

The preferred preparations consumed for nutrition or pleasure containing the thiophenes according to the invention or to be used according to the invention are soups (instant soups, such as onion, leek, asparagus, pea, carrot, tomato, chicken, beef, mushroom, fish, prawn and crab soups, as well as tinned soups with the above taste directions), sauces (instant sauces with the taste directions beef, pork, lamb, fish, crab, prawn, mushroom, asparagus, onion and leek), seasonings (bouillon with the taste directions beef, pork, chicken, lamb and vegetable, as well as various types of seasoning mixes and table salt seasonings), snack foods (baked or deep-fried potato crisps or potato dough products, corn- or peanut-based extrudates), fat- or oil-based products (mayonnaise, remoulade and dressings) and ready meals.

The preparations according to the invention can preferably also be used in seasonings. Suitable seasonings contain e.g. synthetic, natural or nature-identical flavourings as well as carriers such as e.g. maltodextrin, salts such as e.g. common salt, spices, such as e.g. paprika and pepper, saccharides or sugar substitutes or sweeteners, such as e.g. saccharin and flavour enhancers, such as e.g. monosodium glutamate and/or inosine monophosphate.

The preparations according to the invention containing the thiophenes according to the invention or to be used according to the invention can be produced by incorporating the thiophenes according to the invention or to be used according to the invention as a solution or in the form of a mixture with a solid or liquid carrier into the preparations consumed for nutrition or pleasure or used for oral hygiene. Advantageously, the preparations according to the invention that are present as solutions containing the flavour compositions according to the invention can also be converted to a solid preparation by spray drying.

To produce the preparations in another preferred embodiment, the flavour compositions according to the invention and optionally other components of the preparation according to the invention can also be previously incorporated into emulsions, liposomes, e.g. starting from phosphatidylcholine, microspheres, nanospheres or capsules consisting of a matrix suitable for foodstuffs, beverages and tobacco, e.g. starch, starch derivatives, other polysaccharides, natural fats, natural waxes or proteins, e.g. gelatines. Another embodiment consists in the fact that the flavour compositions according to the invention are previously complexed with suitable complexing agents, e.g. with cyclodextrins or cyclodextrin derivatives, preferably β-cyclodextrin, and used in this form.

Other conventional basic, auxiliary and additive substances for foodstuffs, beverages or tobacco can be used as additional components of the preparations consumed for nutrition or pleasure according to the invention, e.g. water, mixtures of fresh or processed, vegetable or animal, basic or raw materials (e.g. raw, fried, dried, fermented, smoked and/or cooked meat, egg, bone, cartilage, fish, crustaceans and molluscs, vegetables, fruit, nuts, vegetable or fruit juices or pastes or mixtures thereof), digestible or non-digestible carbohydrates (e.g. sucrose, maltose, fructose, glucose, dextrins, amylose, amylopectin, inulin, xylans, cellulose), sugar alcohols (e.g. sorbitol, mannitol, xylitol), natural or hydrogenated fats (e.g. tallow, lard, palm fat, coconut fat, hydrogenated vegetable fat), fatty oils (e.g. sunflower oil, groundnut oil, corn oil, thistle oil, olive oil, walnut oil, fish oil, soya oil, sesame oil), fatty acids or salts thereof (e.g. potassium stearate, potassium palmitate), proteinogenic or non-proteinogenic amino acids and related compounds (e.g. taurine, creatine, creatinine), peptides, natural or processed proteins (e.g. gelatines), enzymes (e.g. peptidases, glucosidases, lipases), nucleic acids, nucleotides (inositol phosphate), flavour enhancers (e.g. sodium glutamate, 2-phenoxypropionic acid), emulsifiers (e.g. lecithins, diacylglycerols), stabilisers (e.g. carageenan, alginate, locust bean gum, guar gum), preservatives (e.g. benzoic acid, sorbic acid), antioxidants (e.g. tocopherol, ascorbic acid), chelators (e.g. citric acid), organic or inorganic acidulants (e.g. malic acid, acetic acid, citric acid, tartaric acid, phosphoric acid), bitter substances (e.g. quinine, caffeine, limonin), sweeteners (e.g. saccharin, cyclamate, aspartame, neotame, neohesperidine dihydrochalcone), mineral salts (e.g. sodium chloride, potassium chloride, magnesium chloride, sodium phosphates), substances preventing enzymatic browning (e.g. sulfite, ascorbic acid), essential oils, plant extracts, natural or synthetic dyes or colour pigments (e.g. carotinoids, flavonoids, anthocyans, chlorophyll and derivatives thereof), spices and odorants, synthetic, natural or nature-identical flavourings and tastants.

The preparations according to the invention are preferably used as semi-finished products for the flavouring of preparations made therefrom as finished products.

The invention further provides a process for the production of the thiophenes of formula I according to the invention or to be used according to the invention, with the following step:
reaction of a compound of formula II 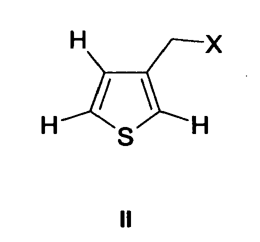
wherein
X represents a leaving group in a nucleophilic substitution reaction, preferably Cl, Br, I, O-tosyl or O-mesyl, particularly preferably Cl,
a) with a C₁ or C₂ - C₄ alkanethiol or a C₁ - C₅ thioalkanoic acid in the presence of a base, preferably an alkali or alkaline earth hydroxide, particularly preferably LiOH, NaOH or KOH,
   or
b) with an alkali or alkaline earth salt, preferably the Li, Na or K salt, of a C₁ or C₂ - C₄ alkanethiol or a C₁ - C₅ thioalkanoic acid.

The thiophenes according to the invention or to be used according to the invention can be present as pure substances or in the form of solutions. As solvents, preferably water, methanol, ethanol, propylene glycol, glycerin, acetone, dichloromethane, diethyl ether, hexane, heptane, triacetin, vegetable oil or fats, such as e.g. vegetable triglyceride (kosher or not), supercritical carbon dioxide or mixtures of the above solvents are used. The shelf life of the pure substance, which is already long at 12 months, can be increased to at least 24 months in solution.

### Examples

Unless otherwise specified, data refer to the weight.

### Synthesis of compounds of formula I:

### Precursor: Synthesis of 3-chloromethylthiophene (compound of formula II with X = Cl)

86 g thionyl chloride were metered into a solution of 65 g thiophen-3-yl methanol (commercially available) in 325 ml chloroform at room temperature. After stirring for one hour, the mixture was hydrolysed with 400 ml iced water, the phases separated and the aqueous phase extracted twice more with chloroform. The combined organic phases were washed with sodium bicarbonate solution and with water and dried over sodium sulfate. After filtering and evaporating the solution *in vacuo,* 75 g of the crude product of 3-chloromethylthiophene were obtained (purity >95%).
a) Representative instructions for preparing the alkyl derivatives of formula I:
   A sodium thiomethylate solution, previously prepared from 11 g sodium and 62 g methanethiol in 500 ml ethanol, was added dropwise to a solution of 75 g chloromethylthiophene (from the precursor) in 600 ml ethanol at 40°C.
   After heating under reflux for one hour, the solution was stirred overnight at room temperature. The solvent was then removed as far as possible *in vacuo* and 400 ml diethyl ether and 400 ml water were added. After separating the phases, the aqueous phase was extracted once with ether. The combined organic phases were dried over sodium sulfate, filtered and the solvent removed *in vacuo.* The residue was fractionally distilled and 50 g 3-(methylthiomethyl)thiophene (compound of formula I with R = CH₃) were obtained (purity >98%).
b) Representative instructions for preparing acyl derivatives of formula I:
   9 g potassium thioacetate are dissolved in 25 ml dimethylformamide and 12 g chloromethylthiophene (from the precursor) are added at room temperature. After the mixture had been stirred overnight at room temperature and for 2 h at 100°C, 25 ml water were added to the cooled solution and extracted three times with MTBE. The organic is washed with sodium bicarbonate and water and dried with sodium sulfate. After filtering, the solvent is removed *in vacuo* and the residue fractionally distilled. 7 g thioacetic acid-S-thiophen-3-yl methyl ester (compound of formula I with R = C(O)CH₃) were obtained (purity >95%).

### Examples of applications for flavour compositions and preparations

### Application example 1: mustard flavour

A typical mustard flavour can be prepared by the following composition:
Mustard oil (100 g), vegetable triglyceride (900 g)
   1.1 Surprisingly, by adding 20 ppm 3-(methylthiomethyl)thiophene, the mustard flavour assumes a stronger, hotter horseradish taste, which also provides the pungent character of horseradish. In addition, a flavour-enhancing effect (booster effect) is observed, which expresses itself as a stronger overall taste impression.
   1.2 A similar effect, milder in its initial intensity, is obtained by adding a mixture of 10 ppm 3-(methylthiomethyl)thiophene and 10 ppm thioacetic acid-S-thiophen-3-yl methyl ester.

The above flavour compositions can be incorporated into mayonnaise, for example, at a dosage of 30 g flavour composition per 100 kg basic mayonnaise preparation.

### Application example 2: garlic flavour

A typical garlic flavour can be prepared by the following composition:
Garlic oil (8.00 g), diallyl sulfide (0.30 g), diallyl disulfide (0.05 g), allyl isothiocyanate (1.00 g), allyl mercaptan (0.10 g) and vegetable triglyceride (990.55 g).
   2.1 Surprisingly, by adding 20 ppm 3-(methylthiomethyl)thiophene, the garlic flavour gains more impact and more freshness. The character of a raw garlic clove becomes more authentic and the oily impression is suppressed.
   2.2 A similar effect, milder in intensity, is caused by the addition of thioacetic acid-S-thiophen-3-yl methyl ester.

The above flavour compositions can be incorporated into a salad dressing, for example, at a dosage of 30 g flavour composition per 100 kg basic salad dressing preparation.

### Application example 3: leek flavour

A typical leek flavour can be prepared by the following composition:
Onion oil (1.00 g), dipropyl disulfide (0.10 g), diallyl disulfide (0.10 g), 3-isopropyl-2-methoxy-pyrazine, 0.1% in triacetin (0.20 g), hexanal (0.30 g), hexanol (0.30 g), cis-3-hexenol (0.50 g), trans-2-hexenal (0.20 g) and vegetable triglyceride (997.30 g).

Surprisingly, by adding 20 ppm 3-(methylthiomethyl)thiophene, the leek flavour obtains a fresher, greener character with slightly hot notes and a spring onion direction.

The flavour composition containing 3-(methylthiomethyl)thiophene can be incorporated in a cream of leek soup, for example, at a dosage of 30 g flavour composition per 100 kg basic cream of leek soup preparation.

## Claims

1. Use of a compound of formula I 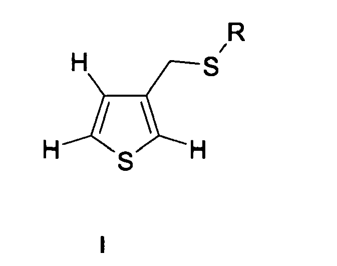 wherein
R represents a C₁ - C₄ alkyl or a C₁ - C₅ acyl residue
as an odorant or flavouring or as a flavour enhancer.

2. Use according to claim 1, wherein R is selected from the group consisting of methyl, ethyl, 1-propyl, 2-propyl, 1-butyl, 2-butyl, 2-methyl-1-propyl-, 2-methyl-2-propyl, formyl, acetyl, propionyl, butyryl, 2-methylpropionyl, valeryl, 2-methylbutyryl and 3-methylbutyryl.

3. Use according to claim 1 or 2, wherein R is methyl.

4. Compound of formula I 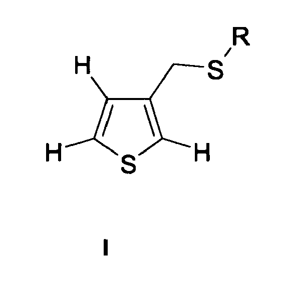 wherein
R represents a C₂ - C₄ alkyl or a C₁ - C₅ acyl residue.

5. Process for the production, enhancement or modification of odour and/or taste properties of a basic composition, comprising the following steps:
- preparation of the basic composition,
- preparation of one or more different compounds of formula I 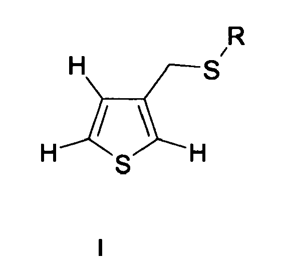
wherein
R represents a C₁ - C₄ alkyl or a C₁ - C₅ acyl residue, and
- mixing the basic composition with a sensorially active quantity of the one compound or of the different compounds of formula I.

6. Flavour composition containing
- a sensorially active quantity of one or more different compounds of formula I 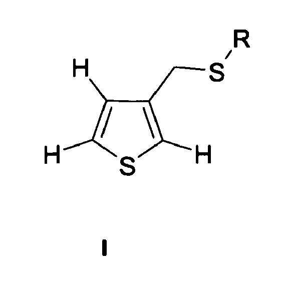
wherein
R represents a C₁ - C₄ alkyl or a C₁ - C₅ acyl residue, and
- a sensorially active quantity of one or more other flavourings,
wherein
the sensorially active quantity of the one or the different compounds of formula I is selected such that the sensory impression created by the sensorially active quantity of the one or more other flavourings is enhanced and/or modified and/or rounded off.

7. Preparation consumed for nutrition or pleasure, containing
(a) a sensorially active quantity of one or more different compounds of formula I 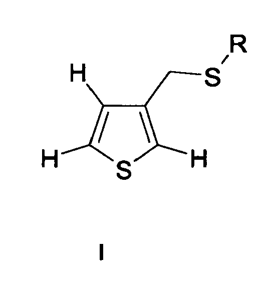 wherein
R represents a C₁ - C₄ alkyl or a C₁ - C₅ acyl residue, or
(b) a flavour composition according to claim 6, and optionally one or more conventional basic, auxiliary or additive substances.

8. Process for the production of a compound of formula I 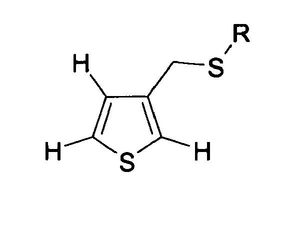 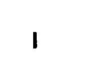 wherein
R represents a C₁ or C₂ - C₄ alkyl or a C₁ - C₅ acyl residue, with the following step:
reaction of a compound of formula II 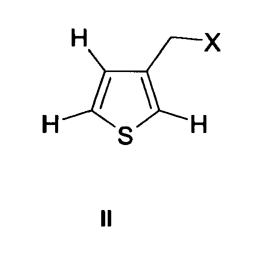
wherein
X represents a leaving group in a nucleophilic substitution reaction,
a) with a C₁ or C₂ - C₄ alkanethiol or a C₁ - C₅ thioalkanoic acid in the presence of a base
or
b) with an alkali or alkaline earth salt of a C₁ or C₂ - C₄ alkanethiol or a C₁ - C₅ thioalkanoic acid.

## Patentansprüche

1. Verwendung einer Verbindung der Formel I 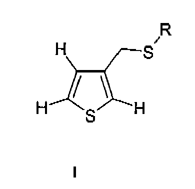 wobei
R einen C₁ - C₄ Alkyl- oder einen C₁ - C₅ Acylrest darstellt
als Riech- oder Aromastoff oder als Aromaverstärker.

2. Verwendung nach Anspruch 1, wobei R ausgewählt ist aus der Gruppe bestehend aus Methyl, Ethyl, 1-Propyl, 2-Propyl, 1-Butyl, 2-Butyl, 2-Methyl-1-propyl-, 2-Methyl-2-propyl. Formyl, Acetyl, Propionyl, Butyryl, 2-Methyl-propionyl, Valeryl, 2-Methyl-butyryl und 3-Methyl-butyryl.

3. Verwendung nach Anspruch 1 oder 2, wobei R Methyl ist.

4. Verbindung der Formel I 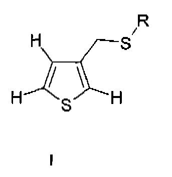 wobei
R einen C₂ - C₄ Alkyl- oder einen C₁ - C₅ Acylrest darstellt.

5. Verfahren zur Erzeugung, Verstärkung oder Modifizierung geruchlicher und/oder geschmacklicher Eigenschaften einer Basiskomposition, umfassend die Schritte:
- Bereitstellen der Basiskomposition,
- Bereitstellen einer oder mehrerer verschiedener Verbindungen der Formel I 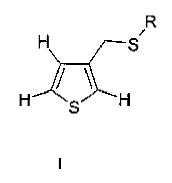 wobei
R einen C₁ - C₄ Alkyl- oder einen C₁ - C₅ Acylrest darstellt, und
- Vermischen der Basiskomposition mit einer sensorisch wirksamen Menge der einen Verbindung oder der verschiedenen Verbindungen der Formel I**.**

6. Aromakomposition, umfassend
- eine sensorisch wirksame Menge einer oder mehrerer verschiedener Verbindungen der Formel I 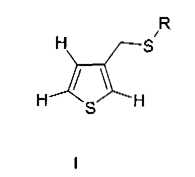
wobei
R einen C₁ - C₄ Alkyl- oder einen C₁ - C₅ Acylrest darstellt, und
- eine sensorisch wirksame Menge eines oder mehrerer anderer
Aromastoffe,
wobei
die sensorisch wirksame Menge der einen oder der verschiedenen Verbindungen der Formel I so gewählt ist, dass der durch die sensorisch wirksame Menge des einen anderen Aromastoffs oder der anderen Aromastoffe vermittelte sensorische Eindruck verstärkt und/oder modifiziert und/oder abgerundet wird.

7. Der Ernährung oder dem Genuß dienende Zubereitung umfassend
(a) eine sensorisch wirksame Menge einer oder mehrerer verschiedener Verbindungen der Formel I 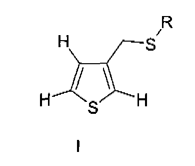 wobei
R einen C₁ - C₄ Alkyl- oder einen C₁ - C₅ Acylrest darstellt oder
(b) eine Aromakomposition nach Anspruch 6,
sowie gegebenenfalls einen oder mehrere übliche Grund-, Hilfs- oder Zusatzstoffe.

8. Verfahren zur Herstellung einer Verbindung der Formel I 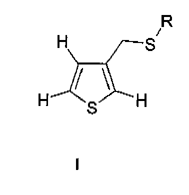 wobei
R einen C₁ oder C₂ - C₄ Alkyl- oder einen C₁ - C₅ Acylrest darstellt, mit folgendem Schritt:
Umsetzen einer Verbindung der Formel II 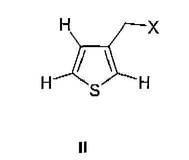
wobei
X eine Abgangsgruppe in einer nucleophilen Substitutionsreaktion darstellt,
(a) mit einem C₁ - C₄ - Alkanthiol oder einer C₁ - C₅ - Thioalkansäure in Gegenwart einer Base
oder
(b) mit einem Alkali- oder Erdalkalisalz eines C₁- oder eines C₂ - C₄ - Alkanthiols oder einer C₁ - C₅ - Thioalkansäure.

## Revendications

1. Utilisation d'un composé de formule I 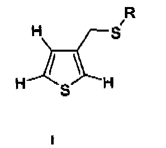 dans laquelle
R représente un alkyle en C₁-C₄ ou un résidu acyle en C₁-C₅
en tant qu'odorant ou aromatisant ou en tant qu'exhausteur de goût.

2. Utilisation selon la revendication 1, dans laquelle R est choisi dans le groupe constitué par méthyle, éthyle, 1-propyle, 2-propyle, 1-butyle, 2-butyle, 2-méthyl-1-propyl-, 2-méthyl-2-propyle, formyle, acétyle, propionyle, butyryle, 2-méthylpropionyle, valéryle, 2-méthylbutyryle et 3-méthylbutyryle.

3. Utilisation selon la revendication 1 ou 2, dans laquelle R est un méthyle.

4. Composé de formule 1 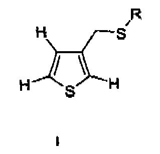 dans laquelle
R représente un alkyle en C₂-C₄ ou un résidu acyle en C₁-C₅.

5. Procédé pour la fabrication, le renforcement ou la modification de propriétés d'odeur et/ou de goût d'une composition basique, comprenant les étapes suivantes:
- préparation de la composition basique,
- préparation d'un ou plusieurs composés différents de formule I 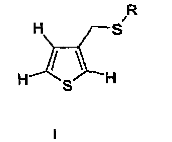
dans laquelle
R représente un alkyle en C₁-C₄ ou un résidu acyle en C₁-C₅, et
- mélange de la composition basique avec une quantité sensoriellement active d'un composé ou des différents composés de formule I.

6. Composition de flaveur, contenant
- une quantité sensoriellement active d'un ou plusieurs composés différents de formule I 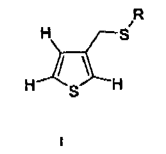
dans laquelle
R représente un alkyle en C₁-C₄ ou un résidu acyle en C₁-C₅, et
- une quantité sensoriellement active d'un ou plusieurs autres aromatisants,
dans laquelle la quantité sensoriellement active du composé ou des différents composés de formule I est choisie de sorte que l'impression sensorielle créée par la quantité sensoriellement active du ou des autres aromatisants est renforcée et/ou modifiée et/ou arrondie.

7. Préparation consommée à des fins de nutrition ou d'agrément, contenant
(a) une quantité sensoriellement active d'un ou plusieurs composés différents de formule I 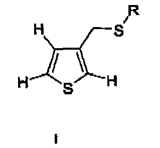 dans laquelle
R représente un alkyle en C₁-C₄ ou un résidu acyle en C₁-C₅, ou
(b) une composition de flaveur selon la revendication 6, et facultativement une
ou plusieurs substances basiques, auxiliaires ou additives classiques.

8. Procédé pour la production d'un composé de formule I 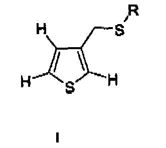 dans laquelle
R représente un alkyle en C₁ ou C₂-C₄ ou un résidu acyle en C₁-C₅, avec l'étape suivante:
réaction d'un composé de formule II 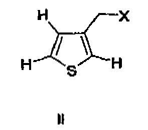
dans laquelle
X représente un groupe partant dans une réaction de substitution nucléophile,
a) avec un alcanethiol en C₁ ou C₂-C₄ ou un acide thioalcanoïque en C₁-C₅, en présence d'une base
ou
b) avec un sel alcalin ou alcalino-terreux d'un alkanethiol en C₂-C₄ ou en C₁
ou d'un acide thioalcanoïque en C₁-C₅.
